# EUROPÄISCHE PATENTANMELDUNG

(11) **EP 1 707 193 A2**
(43) Veröffentlichungstag der Anmeldung: **04.10.2006**
(21) Anmeldenummer: 06090076.8
(22) Anmeldetag: 26.07.2002
(51) Int. Cl.: A61K 31/136, A61K 31/4439, A61P 31/16, A61P 31/14, A61P 31/20, A61K 31/00

(54) **Pharmazeutische Zusammensetzung zur Prophylaxe und/oder Therapie von Viruserkrankungen**

(30) Priorität: 08.08.2001 DE 10138912
(62) Teilanmeldung aus: 02758125.5
(71) Anmelder: MedInnova Gesellschaft für medizinische Innovationen aus akademischer Forschung mbH, 35037 Marburg (DE)
(72) Erfinder: Ludwig, Stephan, Prof. Dr., 48161 Münster (DE); Planz, Oliver, 72108 Rottenburg (DE); Sedlacek, Hans-Harald, 35041 Marburg (DE); Pleschka, Stephan, 35390 Giessen (DE)
(74) Vertreter: Jungblut, Bernhard Jakob

(57) **Zusammenfassung**

Die Erfindung betrifft die Verwendung mindestens einer Wirksubstanz zur Herstellung einer pharmazeutischen Zusammensetzung zur Prophylaxe und/oder Therapie von mindestens einer Viruserkrankung. Sie ist dadurch gekennzeichnet, dass die Wirksubstanz(en) entweder mindestens zwei Kinasen oder mindestens eine SEK Kinase eines zellulären Signalübertragungsweges derart hemmen, dass eine Virusvermehrung gehemmt wird.

## Beschreibung

### Gebiet der Erfindung.

Die Erfindung betrifft die Verwendung mindestens einer Wirksubstanz zur Herstellung einer pharmazeutischen Zusammensetzung zur Prophylaxe und/oder Therapie von mindestens einer Viruserkrankung sowie ein Kombinationspräparat hierfür.

### Hintergrund der Erfindung und Stand der Technik.

Infektionen mit RNA- oder DNA-Viren stellen eine bedeutende Gesundheitsbedrohung für Mensch und Tier dar. Beispielsweise gehören Infektionen mit Influenza-Viren immer noch zu den großen Seuchen der Menschheit und fordern Jahr für Jahr eine Vielzahl an Todesopfern. Sie sind gesamtwirtschaftlich, etwa durch krankheitsbedingte Arbeitsunfähigkeit, ein immenser Kostenfaktor. Von ebenfalls wichtiger wirtschaftlicher Bedeutung sind beispielsweise Infektionen mit dem Borna Disease Virus (BDV), das vor allem Pferde und Schafe befällt, jedoch auch schon beim Menschen isoliert wurde und hier in Zusammenhang mit neurologischen Erkrankungen gebracht wird.

Das Problem der Bekämpfung von insbesondere RNA-Viren ist die durch eine hohe Fehlerrate der viralen Polymerasen verursachte Wandlungsfähigkeit der Viren, die sowohl die Herstellung geeigneter Impfstoffe als auch das Entwickeln von antiviralen Substanzen sehr schwierig macht. Darüberhinaus hat sich gezeigt, dass die Anwendung von antiviralen Substanzen, die direkt gegen Funktionen des Virus gerichtet sind, zwar zu Therapiebeginn eine gute antivirale Wirkung zeigen, aber mutationsbedingt sehr schnell zur Selektion resistenter Varianten führt. Ein Beispiel hierfür ist das anti-Influenza-Agens Amantadin und dessen Derivate, welches bzw. welche gegen ein Transmembranprotein des Virus gerichtet ist bzw. sind. Innerhalb kurzer Zeit nach Anwendung bilden sich resistente Varianten des Virus. Ein weiteres Beispiel sind die neuen Therapeutika für Influenzainfektionen, die das Influenzavirale Oberflächenprotein Neuraminidase hemmen. Hierzu gehört beispielsweise Relanza. In Patienten wurden bereits Relanza-resistente Varianten gefunden (Gubareva et al., J Infect Dis 178, 1257-1262, 1998). Die Hoffnungen, die in dieses Therapeutikum gelegt wurden, konnten somit nicht erfüllt werden.

Aufgrund ihrer meist sehr kleinen Genome und damit verbundener begrenzter Kodierungskapazität für replikationsnotwendige Funktionen sind alle Viren in starkem Maße auf Funktionen ihrer Wirtszellen angewiesen. Durch Einflussnahme auf solche zelluläre Funktionen, die für die virale Replikation notwendig sind, ist es möglich, die Virusreplikation in der infizierten Zelle negativ zu beeinträchtigen. Hierbei besteht für das Virus nicht die Möglichkeit, durch Anpassung, insbesondere durch Mutationen, die fehlende zelluläre Funktion zu ersetzen, um so dem Selektionsdruck zu entweichen. Dies konnte am Beispiel des Influenza A Virus mit relativ unspezifischen Hemmstoffen gegen zelluläre Kinasen und Methyltransferasen bereits gezeigt werden (Scholtissek und Müller, Arch Virol 119, 111-118, 1991).

Es ist bekannt, dass Zellen über eine Vielzahl von Signalübertragungswegen verfügen, mit Hilfe derer auf die Zelle einwirkende Signale in den Zellkern übertragen werden. Dadurch kann die Zelle auf äußere Reize reagieren und mit Zellproliferation, Zellaktivierung, Differenzierung oder kontrolliertem Zelltod reagieren. Diesen Signalübertragungswegen ist gemeinsam, dass sie mindestens eine Kinase enthalten, welche durch Phosphorylierung mindestens ein nachfolgend signalübertragendes Protein aktivieren. Bei Betrachtung der zellulären Prozesse, die nach Virusinfektionen induziert werden, findet man, dass eine Vielzahl von DNA- und RNA-Viren in der infizierten Wirtszelle bevorzugt einen definierten Signalübertragungsweg, den sogenannte Raf/MEK/ERK-Kinase-Signalübertragungsweg aktivieren (Benn et al., J Virol 70, 4978-4985, 1996; Bruder und Kovesdi, J Virol 71, 398-404, 1997; Popik und Pitha, Virology 252, 210-217, 1998; Rodems und Spector, J Virol 72, 9173-9180, 1998). Dieser Signalübertragungsweg ist einer der wichtigsten Signalübertragungswege in einer Zelle und spielt eine bedeutende Rolle in Proliferations- und Differenzierungsprozessen. Wachstumsfaktorinduzierte Signale werden dabei durch sukzessive Phosphorylierung von der Serin/Threonin Kinase Raf auf die Dualspezifische Kinase MEK (MAP Kinase Kinase/ERK Kinase) und schließlich auf die Kinase ERK (extracellular signal regulated kinase) übertragen. Während man als Kinase-Substrat für Raf nur MEK kennt und für MEK als einzige Substrate die ERK-Isoformen identifiziert wurden, kann ERK eine ganze Reihe an Substraten phosphorylieren. Hierzu gehören beispielsweise Transkriptionsfaktoren, wodurch die zelluläre Genexpression direkt beeinflusst wird (Cohen, Trends in Cell Biol 7, 353-361, 1997; Robinson und Cobb, Curr.Opin.Cell Biol 9, 180-186, 1997, Treisman, Curr.Opin Cell Biol 8, 205-215, 1996). Die Untersuchung der Rolle dieses Signalübertragungsweges in zellulären Entscheidungsprozessen hat zur Identifizierung mehrerer pharmakologischer Inhibitoren geführt, welche den Signalübertragungsweg unter anderem auf der Ebene von MEK, also relativ am Beginn des Signalübertragungsweges hemmen (Alessi et al., J Biol Chem 270, 27489-27494, 1995; Cohen Trends in Cell Biol 7, 353-361, 1997; Dudley et al., PNAS USA 92, 7686-7689, 1995; Favata et al., J Biol Chem 273, 18623-18632, 1998).

Neuere Daten zeigen, dass die Inhibition des Ras-Raf-MEK-ERK-Signalübertragungsweges durch Wirksubstanzen, welche relativ selektiv eine der an diesem Signalübertragungsweg beteiligten Kinasen, insbesondere die MEK inhibieren, die intrazelluläre Vermehrung von intranukleär replizierenden Negativstrang-Viren, beispielsweise von Influenza A Virus und Borna Disease Virus (BDV) drastisch hemmen kann (Pleschka et al., Nature Cell Biol 3, 301-305, 2001; Planz et al., J Virol 10, 4871-4877, 2001).

Der Nachteil bekannter antiviraler Wirksubstanzen ist, dass sie entweder gegen eine virale Komponente gerichtet sind und so schnell zu Resistenzen führen (s. Amantadin), oder gegen zelluläre Faktoren zu breit und unspezifisch wirken (Bsp. Methyltransferaseinhibitoren), wobei mit großen Nebenwirkungen zu rechnen ist. Entsprechend ist von keiner der gegen zelluläre Faktoren wirkenden Substanzen bekannt, dass sie bislang zu einem Therapeutikum für Viruserkrankungen entwickelt worden wäre. Andererseits kann die Inhibition anderer Kinasen, beispielsweise die Inhibition der Kinase JNK des MEKK/SEK/JNK Signalübertragungsweges, die Virusvermehrung verstärken.

Weiterhin ist bekannt, dass die verstärkte Aktivierung wiederum anderer Kinasen, beispielsweise der Proteinkinase C (PKC), die Replikation von Viren hemmt (Driedger und Quick, WO 92/02484).

Bislang bestand die Vorstellung, dass Negativstrang-RNA Viren nur den Raf-MEK-ERK- Signalübertragungsweg für ihre Vermehrung nutzen und demzufolge eine Hemmung der Virusvermehrung für diesen Signalübertragungsweg, insbesondere durch eine Inhibition von MEK, zu einer vollständigen Hemmung der Virusvermehrung führt. Da in einer Zelle die Signalübertragungswege jedoch kaum eine in sich abgeschlos- sene Funktionen aufweisen, sondern bei Aktivierung des einen Signalübertragungsweges über Kreuzvernetzungen weitere Signalübertragungswege in unterschiedlichem Ausmaß zusätzlich aktiviert werden, ist grundsätzlich nicht auszuschließen, dass der Raf-MEK-ERK-Signalübertragungsweg sowohl von der Zelle als auch von den Viren umgangen werden kann und der therapeutische Effekt einer Wirksubstanz, die eine Virusvermehrung für den Raf-MEK-ERK-Signalübertragungsweg hemmt, hierdurch eingeschränkt werden könnte.

### Technisches Problem der Erfindung.

Daher besteht ein großer Bedarf für das Auffinden und Verwenden antiviraler Wirksubstanzen, welche zusätzlich oder ergänzend zu solchen wirken, die die Virusvermehrung bei dem Raf-MEK-ERK-Signalübertragungsweg hemmen. Das Auffinden solcher Wirksubstanzen wird dadurch erschwert, dass beispielsweise Inhibitoren von Kinasen außerhalb des Raf-MEK-ERK-Signalübertragungsweges, beispielsweise Inhibitoren der Kinase JNK, eine Virusvermehrung fördern können, also die gegenteilige als die gewünschte Wirkung erzielen (Ludwig et al., J Biol Chem 276, 1-9, 2001), und dass wiederum die Aktivierung ausgewählter Kinasen, beispielsweise PKC, durch Substanzen antiviral wirken kann.

### Grundzüge der Erfindung und bevorzugte Ausführungsformen.

Ein Gegenstand der vorliegenden Erfindung ist die Verwendung mindestens einer Wirksubstanz zur Prophylaxe und/oder Therapie von mindestens einer Viruserkrankung, wobei die Wirksubstanz(en) entweder auf mindestens zwei Kinasen eines zellulären Signalübertragungsweges derart einwirkt bzw. einwirken, dass eine Virusvermehrung im wesentlichen gehemmt wird oder eine SEK Kinase im wesentlichen hemmt bzw. hemmen. Gegenstand der vorliegenden Erfindung ist des weiteren die Kombination mindestens einer erfindungsgemäßen Wirksubstanz mit mindestens einem weiteren antiviral wirkenden Wirkstoff, welcher vorzugsweise kein Kinaseinhibitor ist, für die Prophylaxe und/oder Therapie einer Virusinfektion.

Es wurde nun überraschenderweise gefunden, dass die Inhibition einer SEK Kinase im MEKK/SEK/JNK-Signalübertragungsweg die Virusvermehrung hemmt. Dies ist deswegen so überraschend, da die Hemmung von JNK im MEKK/SEK/JNK-Signalübertragungsweg das Gegenteil, eine verstärkte Virusvermehrung, bewirkt (Ludwig et al., J Biol Chem 276, 1-9, 2001). Da der JNK-AP-1 Signalweg wesentlich an der Virus-induzierten Expression von IFNß, einem stark antiviral wirksamen Cytokin, beteiligt ist, scheint die fehlende IFNß Expression nach Hemmung von JNK eine verstärkte Virusvermehrung zu ermöglichen. Weiterhin wurde überraschenderweise gefunden, dass eine Hemmung von mindestens zwei Kinasen eines zellulären Signalübertragungsweges durch mindestens eine Wirksubstanz die Virusvermehrung weitaus stärker hemmt als es der Fall ist, wenn nur eine Kinase durch einen Wirkstoff gehemmt wird. So führt beispielsweise die Inhibition von p38 und MAPKAPK3 oder die Inhibition von MKK6 und p38 oder die Inhibition von Raf und MEK zu einer deutlich stärkeren antiviralen Wirkung als die Inhibition nur einer, insbesondere der (die zweite Kinase) aktivierenden, Kinase. Es wurde des weiteren überraschend gefunden, dass die Hemmung von mindestens einer Kinase eines zellulären Signalübertragungsweges durch mindestens eine Wirksubstanz und die zusätzliche Verabreichung eines antiviralen Wirkstoffes, welcher kein Inhibitor einer Kinase darstellt, zu einer starken Hemmung der Virusvermehrung führt. Diese Hemmung durch die Kombination war stärker als bei Addition der Einzelwirkungen der Komponenten dieser Kombination zu erwarten gewesen wäre. So wurden beispielweise Zellen mit Influenza A infiziert und die Zellen mit dem Kinaseinhibitor U0126, von welchem bekannt ist, dass er im Raf/MEK/ERK Signalübertragungsweg die MEK Kinase inhibiert und zugleich mit Amantadin behandelt. Von Amantadin ist bekannt, dass es nicht bei allen Influenza A Viren und nicht bei Infuenza B Viren wirkt, des weiteren, dass es in kurzer Zeit zur Bildung von resistenten Virusvarianten führt. Die Kombination von U0126 und Amantadin führte zu einer Hemmung der Vermehrung von Influenza A Viren, wobei die Kombination stärker wirken kann als die beiden einzelnen Komponenten allein.

Zu diesen zellulären Signalübertragungswegen gehören beispielsweise: MEKK2,-3/MEK5/ERK5; Raf/MEK/ERK; MEKK/SEK/JN; JAK1, JAK2, JAK3, TYK2/ Hetero- und Homodimere von JAK1,-2,-3,TYK2; ASK/MKK3,-6/p38; ASK/MKK4,-7/JNK; MEKK4/ MKK4,-7; DLK/MKK4,-7; Tpl-2/MKK4,-7; Tpl-2/MEK5/ERK5; MLK-3/ MKK3,-6; MLK-3/ MKK4,-7; TAK/NIK/IKK; TAK/MKK3,-6; TAK/MKK4,-7; PAK/MKK3,-6; PAK/IKK; Cot,Tpl-2/IKK; PKC/IKK; PKB/IKK; PKC/Raf; PAK/Raf; Lck/Raf; MEKKs/IKK; PI3K/PDK1/ PKB; JAK/TYK/PLCgamma; JAK/Tyk/Stat Komplexe; MAP Kinasen/MAPKAP-Kinasen, beispielsweise: ERK/3pK, ERK/Rskp90, p38/MAPKAP-Kinasen, p38/ 3pK.

Einzelheiten zu diesen Signalübertragungswegen wurden von Sedlacek (Drug 59, 435-476, 2000) und in verschiedenen anderen Veröffentlichungen beschrieben (Karin and Ben-Neriah, Ann Rev Immunol 18, 621-663, 2000; Vertegaal et al., Cell Signal 12, 759-768, 2000; Vanhaesebroeck and Alessi, Biochem J 346,,561-576, 2000; Ludwig et al., Mol Cell Biol 16, 6687-6697, 1996; New et al., J Biol Chem 274, 1026-1032, 1999; Ni et al., Biochem Biophys Res Commun, 243, 492-496, 1998; Rebecchi and Pentyala, Physiol Rev 80, 1291-335, 2000; Cohen, Trends in Cell Biol 7, 353-361, 1997; Robinson und Cobb, Curr Opin Cell Biol 9, 180-186, 1997; Rane and Reddy, Oncogene, 19, 5662-5679, 2000; Sun et al., Curr Biol. 10, 281-284, 2000; Garrington and Johnson, Curr Opin Cell Biol 11, 211-218, 1999).

Wirksubstanz im Sinne der vorliegenden Erfindung ist eine Substanz, die in der Lage ist, entweder auf mindestens eine Kinase eines zellulären Signalübertragungsweges derart einzuwirken, dass eine Virusvermehrung im wesentlichen gehemmt ist oder eine SEK Kinase eines zellulären Signalübertragungsweges im wesentlichen zu hemmen. Weiterhin sind als Wirksubstanzen im Sinne dieser Erfindung Derivate der Wirksubstanzen zu verstehen, die beispielsweise durch enzymatische Spaltung in eine erfindungsgemäße aktive Wirksubstanz umgewandelt werden. Wirksubstanzen im Sinne der vorliegenden Erfindung sind außerdem Vorstufen von Wirksubstanzen, welche metabolisch in eine erfindungsgemäß aktive Substanz umgewandelt werden. Zu den Wirksubstanzen im Sinne der vorliegenden Erfindung gehören beispielsweise: Kinase-inhibierende Flaven-Derivate oder Benzpyran-Derivate; Kinase-inhibierende Derivate des 4H-1-Benzopyran, beispielsweise wie in EP 0137193 und EP 366061 offenbart. Die Struktur-Wirkungsbeziehung für diese Derivate wurde beispielsweise von Sedlacek et al. (Int J Oncol 9, 1143-1168, 1996) im Detail beschrieben; Flavopiridolderivate, beispielsweise von Kim et al., J Med Chem 43(22), 4126-4134, 2000 offenbart, insbesondere Thio-Flavopiridol und Oxy-Flavopiridol; 2-(2-Amino-3-methoxyphenyl)-4-oxo-4H-(1) benzopyran, beispielsweise von Ebendal (WO 00/50030) offenbart; 7,12-Dihydro-indolo (3,2-d)(1) Benzazepin-6(5H)-one (NSC 664704 Sausville et al. Pharmacol. Ther. 82(2-3), 285-292, 1999); Phosphokinaseinhibitoren, beispielsweise 7OH-Staurosporine und ihre Phosphokinase inhibierenden Derivate, Butyrolactone, Roscovitine, Purvalanol A, Emodin, Anilinoquin-azoline (PD-168393 und PD 169414), PD 184352 (Duesbery et al., Nature Medicine 5(7), 736-737, 1999) und Phenylamino-pyrimidine (STI 571, CGP 78850, CP 358774, CP59326 und CGP 60474)), Trioylimidazole (1-779450) (Meijer et al., Parmacol.Ther.82(2-3), 297-284, 1999; Sedlacek et al., Int J Oncol 9, 1143-1168, 1996; Sedlacek Drug 59(3), 435-476, 2000); Paullone (Zaharevitz et al., Cancer Res.59, 2566-2569, 1999); SB203580 [4-(4-Fluorophenyl)-2-(4-methylsulfinylphenyl)-5-(4-pyridy 1)1H-imidazol, (Ishikawa et al., J Neurochem 75(2), 494-502, 2000, Harada und Sugimoto, Jpn J Pharmacol 79(3), 369-378, 1999)]; Kinase-inhibierende Buatadien-Derivate, insbesondere Derivate von Diaminodicyano-(R)-thiobutadien; U0126 [1,4-Diamino-2,3-dicyano-1,4-bis(2aminophenylthio) butadien] (Favata et al., J Biol Chem 273(29), 18623-18632, 1998; DeSilva et al., J Immunol 160, 4175-4181, 1998)]; PD098059 [2-2'-amino-3'-methoxyphenyl)-oxanaphtalen-4-on)], (Dudley et al., PNAS USA 92, 7686-7686, 1995); PD184352 [2-(2-chlor-4-jod-phenylamino)-N-cyclo-propylmethoxy-3,4-difluorbenzamid], (Sebolt-Leopold et al., Nature Med 5, 810-816, 1999); 3-Aminomethylen-indolin-Derivate (Heckel et al., DE 92 4401, Eberlein et al., DE 84 4003; WO 00/018734); CEP-1347 (KT7515) bisethylthiomethyl (Maroney et al., J Neurochem 73(5), 1901-1912, 1999); Tetrapyrrolische Makrocyclen, beispielsweise 5,10,15,20-Tetraarylporphyrin und 5,10,15-Triarylcorrol (Aviezer et al., WO 00/27379); Pyrimidonderivate (Ando et al., WO 00/018758); 3-Aminomethylen-indolin-Derivate (Heckel et al., DE 92 4401, Eberlein et al., DE 84 4003; WO 00/018734); Pyrazolo (3,4-b) pyridin- Derivate (Bursuker et al., WO 99/30710); Pyrazol-Derivate (Anantanarayan et al., WO 00/031063); 1,4-substituierte Piperidinderivate (Caravatti et al., EP 374095); lipoide Ammoniumsalze (Daniel et al., WO 90/04918); 4-(4-Fluorophenyl)-2-(4-Hydroxyphenyl)-5-(4-Pyridyl)1H-Imidazol (SB202190);SL 327 (ein Butadienderivat); Dominant negative Mutanten einer Kinase eines zellulären Signalübertragungsweges; Antisense-Oligonukleotide, welche sich spezifisch an die DNA-Sequenz oder mRNA Sequenz kodierend für eine Kinase eines zellulären Signalübertragungsweges anlagern und deren Transkription oder Translation inhibieren; dsOligonukleotide, die geeignet sind zur gezielten Degradierung der mRNAs von Kinasen eines zellulären Signalübertragungsweges durch die RNAi-Technologie entsprechend der Methode, wie von Tuschl et al (Genes Dev 13: 3191-3197, 1999) und von Zamore et al (Cell 101: 25-33, 2000) beschrieben; Antikörper oder Antikörperfragmente spezifisch für eine Kinase eines zellulären Signalübertragungsweges, oder Fusionsproteine, enthaltend mindestens ein Antikörperfragment, beispielsweise ein Fv-Fragment, welche die Kinaseaktivität mindestens einer Kinase inhibieren; Peptide, welche die Interaktion von mindestens zwei Kinasen eines zellulären Signalübertragungsweges inhibieren.

Bevorzugt erfolgt die Verwendung mindestens einer erfindungsgemäßen Wirksubstanz bei Viruserkrankung, die durch RNA- oder DNA-Viren, vorzugsweise Negativstrang-RNA Viren, beispielsweise Influenza-Viren, oder Borna-Viren, hervorgerufen wird.

Eine weitere Ausführungsform der vorliegenden Erfindung betrifft ein Kombinationspräparat zur Prophylaxe und/oder Therapie von mindestens einer Viruserkrankung, enthaltend mindestens zwei Wirksubstanzen, welche entweder auf mindestens zwei Kinasen eines zellulären Signalübertragungsweges derart einwirken, dass eine Virusvermehrung im wesentlichen gehemmt wird oder eine SEK Kinase im wesentlichen hemmen, vorzugsweise ausgewählt aus bereits vorstehend aufgeführten Wirksubstanzen, wobei das Kombinationspräparat in Form eines Gemisches oder als einzelne Komponenten zur gleichzeitigen oder zeitlich unterschiedlichen Anwendung an gleichen oder unterschiedlichen Orten anwendbar ist.

Durch die Verabreichung eines Kombinationspräparats, enthaltend mindestens zwei unterschiedliche Wirksubstanzen, wird die Virusvermehrung durch Einwirken auf mindestens zwei Kinasen eines zellulären Signalübertragungswegs gehemmt. Die Verabreichung des Kombinationspräparates kann als Mischung der Wirksubstanzen erfolgen. Die Wirksubstanzen können pro Verabreichung jedoch auch getrennt voneinander an dem gleichen Ort, beispielsweise intravenös, oder auch an voneinander getrennten Orten, gleichzeitig oder auch zu unterschiedlichen Zeitpunkten innerhalb eines Zeitraumes, in welchem die zuerst verabreichte Substanz noch Wirkung zeigt, beispielsweise in einem Zeitraum von drei Tagen, verabreicht werden.

Eine weitere Ausführungsform dieser Erfindung stellt die Verabreichung eines Kombinationspräparates dar aus mindestens einer erfindungsgemäßen Wirksubstanz und mindestens einem antiviralem Wirkstoff, welcher kein Kinaseinhibitor darstellt. Zu diesen antiviralen Wirkstoffen gehören beispielsweise: 1-Adamantanamin (Amantadin); Rimantadine; Neuraminidase-Inhibitoren wie beispielsweise Relenza; synthetische Nucleosidanaloga wie beispielweise 3-Deaza adenosin und Ribavirin.

Eine weitere Ausführungsform der vorliegenden Erfindung betrifft ein Testsystem zum Auffinden von Wirksubstanzen, welche entweder auf mindestens zwei Kinasen eines zellulären Signalübertragungsweges oder auf eine SEK Kinase derart hemmen, dass eine Virusvermehrung im wesentlichen gehemmt wird enthaltend: a. mindestens eine mit mindestens einem Virus infizierbare Zelle, die entweder mindestens zwei Kinasen eines zellulären Signalübertragungsweges oder mindestens eine SEK-Kinase enthält und mindestens einen die Zellen infizierenden Virus oder b. mindestens eine mit mindestens einem Virus infizierte Zelle, die entweder mindestens zwei Kinasen eines zellulären Signalübertragungsweges oder mindestens eine SEK-Kinase enthält.

Zellen im Sinne der vorliegenden Erfindung sind Zellen aus unterschiedlichen Organen und Geweben, beispielsweise Zellen der Blut- und Lymphgefäße, Zellen, die Körperhöhlen auskleiden. Ebenfalls umfasst sind Zellkulturen, besonders solche, welche von Zellbanken, beispielsweise der ATCC, zu erwerben sind, insbesonders permissive, eukaryotische Zellkulturen, beispielsweise: 293, 293T und 293T7 (*Homo sapiens*); B82, NIH 3T3, L929 aus *Mus musculus;* BHK aus *Cricetus cricetus;* CHO aus *Cricetulus griseus;* MDCK aus *Canis familiaris;* Vero, COS-1 und COS-7 aus *Cercopithecus aethiops;* sowie primäre Embryo-Fibroblasten aus *Gallus gallus* (CEF cells).

Beispielsweise wird in dem erfindungsgemäßen Testsystem zum Auffinden von Wirksubstanzen durch Zugabe von Substanzen, vorzugsweise in Konzentrationen von 0.001µMol bis 100 µMol, und Viren in einer Partikelzahl, welche die ausgewählte Zelle infizieren können, geprüft, ob eine Substanz in der Lage ist, die Virusvermehrung zu hemmen, ohne die Zelle zu schädigen.

Vorzugsweise handelt es sich bei dem in dem erfindungsgemäßen Testsystem verwendeten Virus um ein RNA- oder DNA-Virus, vorzugsweise um ein Influenza-Virus.

In einer bevorzugten Ausführungsform enthält die Zelle des erfindungsgemäßen Testsystems mindestens eine überexprimierte Kinase, insbesondere durch Einführung eines oder mehrerer die Kinase(n) kodierenden Gens bzw. Gene. Durch diese Überexpression werden Substanzen entdeckt, welche Kinasen sowohl stark inhibieren wie auch intrazellulär zur Inhibition der überexprimierten Kinase eine hohe Konzentration erreichen können. Zur Kontrolle ist in einer Zelle des erfindungsgemäßen Testsystems die Expression für mindestens eine Kinase inhibiert, beispielsweise: durch die Einführung einer antisense DNA oder einer antisense RNA; durch die Einführung mindestens eines Genes kodierend für mindestens eine dominantnegative Mutante mindestens einer übergeordneten Kinase; und/oder durch die Einführung mindestens eines Genes kodierend für mindestens eine dominant negative Mutante mindestens einer untergeordneten Kinase eines Signalübertragungsweges.

Eine weitere Ausführungsform der vorliegenden Erfindung betrifft ein Verfahren zum Auffinden von mindestens einer Wirksubstanz zur Prophylaxe und/oder Therapie von Viruserkrankungen, welche die Vermehrung von Viren bei Viruserkrankungen im wesentlichen hemmt bzw. hemmen, enthaltend folgende Schritte: a. Inkontaktbringen mindestens eines erfindungsgemäßen Testsystems mit mindestens einer potentiellen Wirksubstanz, und b. Bestimmung der Auswirkung auf die Virusvermehrung.

Inkontaktbringen im Sinne dieser Erfindung kann beispielsweise durch Zugabe der Wirksubstanzen in das Nährmedium einer Zellkultur oder durch lokale oder systemische Verabreichung der Wirksubstanzen in einen Organismus erfolgen. Inkontaktbringen im Sinne der vorliegenden Erfindung umfasst ebenfalls die nach dem Stand der Technik üblichen Verfahren, die das Einführen von Substanzen in intakte Zellen erlauben, beispielsweise Infektion, Transduktion, Transfektion und/oder Transformation und weitere dem Fachmann bekannte Methoden. Diese Verfahren sind insbesondere bevorzugt, wenn es sich bei der Substanz um Viren, nackte Nukleinsäuren, beispielsweise antisense DNA und/oder antisense RNA, Viroide, Virosomen und/oder Liposomen handelt, wobei Virosomen und Liposomen ebenfalls geeignet sind, neben einem Nukleinsäuremolekül weitere Wirksubstanzen in die Zelle zu bringen.

Das Bestimmen der Auswirkung auf die Virusvermehrung erfolgt beispielsweise mittels Plaque-Assays durch Vergleich von Virustiter infizierter und nichtinfizierter Zellen.

Eine weitere bevorzugte Ausführungsform der vorliegenden Erfindung betrifft ein Verfahren zur Herstellung eines Arzneimittels zur Prophylaxe und/oder Therapie von mindestens einer Viruserkrankung, welche die Vermehrung von Viren bei Viruserkrankungen im wesentlichen hemmt bzw. hemmen, enthaltend folgende Schritte: a. Durchführen eines erfindungsgemäßen Testsystems und b. Versetzen des/der aufgefundenen Wirksubstanz/en mit mindestens einem Hilfs- und/oder Zusatzstoff.

Vorzugsweise wird die Wirksubstanz gemäß vorliegender Erfindung für die lokale oder systemische Verabreichung in einen Organismus mit Hilfe der dem Fachmann geläufigen Methoden und Hilfs- und/oder Zusatzstoffen zu einem Arzneimittel zubereitet.

Geeignete Hilfs- und Zusatzstoffe, die beispielsweise der Stabilisierung oder Konservierung des Arzneimittels oder Diagnostikums dienen, sind dem Fachmann allgemein bekannt (siehe z. B. Sucker H et al. (1991) Pharmazeutische Technologie, 2. Auflage, Georg Thieme Verlag, Stuttgart). Beispiele für solche Hilfs- und/oder Zusatzstoffe sind physiologische Kochsalzlösungen, Ringer-Dextrose, Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Die lokale Verabreichung kann beispielsweise auf die Haut (beispielsweise mit einem transdermalen System), auf die Schleimhaut, in eine Körperhöhle, in ein Organ (z.B. i.m.), in ein Gelenk oder in das Binde- oder Stützgewebe erfolgen. Die systemische Verabreichung erfolgt vorzugsweise in den Blutkreislauf, beispielsweise i.v., in die Peritonealhöhle oder in die Bauchhöhle.

Die Arzneimittelzubereitung enthaltend die erfindungsgemäße Wirksubstanz richtet sich nach der Art der Wirksubstanz und der Art ihrer Verabreichung und kann beispielsweise eine Lösung, eine Suspension, eine Salbe, ein Puder, eine Sprayform oder eine andere Inhalationszubereitung darstellen. Vorzugsweise werden Nukleotidsequenzen mit dem Fachmann bekannten Methoden in einen viralen Vektor oder ein Plasmid eingefügt und mit Hilfsstoffen für die Zelltransfektion versetzt. Zu diesen Hilfsstoffen gehören beispielsweise kationische Polymere oder kationische Lipide. Antisense-Oligonukleotide werden mit den dem Fachmann geläufigen Methoden derivatisiert, um sie vor dem enzymatischen Abbau durch DNAsen oder RNAsen zu schützen.

Die Wirksubstanz gemäß der Erfindung kann in Form eines Salzes, Esters, Amids oder als eine Vorstufe vorliegen, wobei vorzugsweise nur Modifikationen der Wirksubstanz verwendet werden, die keine übermäßige Toxizität, Irritationen oder allergische Reaktionen am Patienten auslösen.

Die Wirksubstanz wird unter sterilen Bedingungen, mit einem physiologisch akzeptablen Trägerstoff und möglichen Preservativen, Puffern oder Treibmitteln, je nach Bedarf, vermischt. Derartige Trägerstoffe für Arzneimittelzubereitungen sind dem Fachmann geläufig.

Bevorzugt wird die erfindungsgemäße Wirksubstanz in einer einmaligen Dosis, besonders bevorzugt in mehreren Dosen verabreicht, wobei die einzelnen Dosen die maximal tolerable Dosis (MTD) der jeweiligen Wirksubstanz für den Menschen nicht übersteigt. Vorzugsweise wird eine Dosis gewählt, welche die Hälfte der MTD beträgt. Beispielsweise liegt für die Infusion von Flavopiridol die MTD beim Tumorpatienten bei 50mg/m²/ dx3 (Senderowicz et al J Clin Oncol 16(9): 2986-2999,1998). Für die Anwendung am Menschen im Sinne der vorliegenden Erfindung ist Flavopiridol somit in einer täglichen Dosis von 0,1- 50mg/m², vorzugsweise von 5-30 mg/m², besonders bevorzugt von 25mg/m² zu verabreichen. Die Tagesdosis kann einmalig am Tag oder in mehreren Teilportionen aufgeteilt über den Tag hinweg, vorzugsweise in etwa gleichen Zeitabständen verabreicht werden.

Gemäß der vorliegenden Erfindung kann die Verabreichung entweder lokal oder systemisch, nur an einem Tag oder über mehrere Tage täglich oder an jedem zweiten oder dritten Tag über mehrere Wochen erfolgen, bis eine therapeutische Wirkung sichtbar wird.

Weitere Varianten der Erfindung sind in den Ansprüchen 17 bis 19 angegeben, wobei hierfür die vorstehenden und nachfolgenden Ausführungen analog gelten.

### Beispiel 1: Positivkontrolle I (Influenza A Virus)

Für die Vermehrung von Influenza A-Viren werden permissive, eukaryontische Zellkulturen (Madine darby canine kidney (MDCK)-Zellen), in parallelen Ansätzen mit gleicher Zellzahl, nach allgemeinen für Zellkulturen üblichen Methoden, mit einer physiologischen Salzlösung gewaschen und mit einer gleichen Menge des infektiösen Influenza A Virusstammes WSN-HK (Reassortante mit sieben Gensegmenten des Influenza-Stamms A/WSN/33 und dem NA-Gen des Influenza-Stamms A/HK/8/68), in einem Verhältnis von 0,0025 infektiöse Viruspartikel pro Zelle für eine Stunde bei Raumtemperatur, infiziert. 30 min vor der Infektion werden die MDCK-Zellen in geeignetem Zellkulturmedium, welches zur positiven Kontrolle in unterschiedlichen Konzentrationen mit dem Kinase-Inhibitor U0126 [1,4-diamino-2,3-dicyano-1,4-bis(2aminophenylthio)butadien] versetzt ist (0µM, 30µM, 40µM, 50µM gelöst in DSMO) bei 37°C und 5% CO2-Konzentration, inkubiert. Als Lösungsmittelkontrolle werden MDCK-Zellen mit Zellkulturmedium inkubiert, welches mit entsprechenden unterschiedlichen Mengen an DMSO versetzt ist. Während der Infektion wird dem Inokulum der Kinase-Inhibitor U0126 bzw. als Lösungsmittel DMSO in den entsprechenden Konzentrationen zugegeben. Anschließend wird das Inokulum entfernt und die infizierten Zellen werden in geeignetem Zellkulturmedium, welches in unterschiedlichen Konzentrationen mit dem Kinase-Inhibitor U0126 versetzt ist (0µM, 30µM, 40µM, 50µM gelöst in DMSO), für 48h, bei 37°C und 5% CO₂-Konzentration, inkubiert. Als Lösungsmittelkontrolle werden infizierte MDCK-Zellen mit Zellkulturmedium inkubiert, welches mit entsprechenden unterschiedlichen Mengen an DMSO versetzt ist. 24 Stunden nach der Infektion werden 200µl des Mediumüberstandes entnommen und das gleiche Volumen an Inhibitor- bzw. DMSOhaltigem Zellkulturmedium dem Mediumüberstand wieder zugegeben. Nach 48 Stunden wird erneut eine Probe entnommen. Die Zellkulturüberstände der jeweiligen Proben für den 24- und den 48- Stunden-Wert werden nach üblichen virologischen Methoden auf die Menge an haemagglutinierenden Einheiten (HA-Titer), welche die Gesamtproduktion an Viruspartikeln darstellt, und auf die Menge an neu gebildeten infektiösen Viruspartikeln (Plaque-Assay auf MDCK-Zellen) hin untersucht. Im Ergebnis kann in einem solchen experimentellen Ansatz festgestellt werden, dass es bei zunehmender Konzentration des Kinase-Inhibitors U0126 im Zellkulturmedium zu einer signifikanten Reduktion (ca. 80% bei 50µM U0126) der Anzahl von neu gebildeten, infektiösen Viruspartikeln, im Vergleich zum Kontrollansatz ohne Inhibitor U0126 bzw. den Lösungsmittelkontrollen kommt. Die makroskopische Untersuchung von MDCK-Zellen, welche mit entsprechenden Konzentrationen an DMSO, bzw. in DMSO gelöstem Inhibitor U0126 behandelt wurden, als auch eine Zytotoxizitätsuntersuchung mittels Propidiumjodidfärbung, zeigt, daß weder Lösungsmittel noch Inhibitor einen signifikanten zytotoxischen Effekt auf die Zellen haben.

### Beispiel 2: Positivkontrolle II (Influenza B Virus)

Dieses Beispiel zeigt, daß bei einer Konzentration von 60µM des MEK-Inhibitors U0126 im Zellkulturmedium auch die Anzahl der neu gebildeten, infektiösen Influenza B Viruspartikel signifikant reduziert ist. Für die Vermehrung von Influenza B Viren werden permissive, eukaryontische Zellkulturen (Madine darby canine kidney (MDCK)-Zellen), in parallelen Ansätzen mit gleicher Zellzahl, nach allgemein üblichen Methoden für die Zellkultur, mit einer physiologischen Salzlösung gewaschen und mit einer gleichen Menge des infektiösen Influenza B Virusstammes Massachusetts/6/93, in einem Verhältnis von 0,01 infektiöse Viruspartikel pro Zelle für eine Stunde bei Raumtemperatur, infiziert. Nach der Infektion wird das Inokulum entfernt und die infizierten Zellen in geeignetem Zellkulturmedium (enthält 2µg/ml Trypsin), welches mit dem MEK-Inhibitor U0126 versetzt ist (60µM gelöst in DMSO), für 60h, bei 37°C und 5% CO₂-Konzentration, inkubiert. Als Kontrolle werden infizierte MDCK-Zellen mit Zellkulturmedium inkubiert, welches mit der entsprechenden Menge an DMSO versetzt ist. Alle 12 Stunden nach der Infektion werden Proben des Mediumüberstandes entnommen. Die jeweiligen Proben der Zellkulturüberstände werden nach üblichen virologischen Methoden auf die Menge an neu gebildeten infektiösen Viruspartikeln (Plaque-Assay auf MDCK-Zellen) hin untersucht. Im Ergebnis kann in einem solchen experimentellen Ansatz festgestellt werden, daß es bei der entsprechenden Konzentration des MEK-Inhibitors U0126 im Zellkulturmedium zu einer signifikanten Reduktion (ca. 90%) der Anzahl von neu gebildeten, infektiösen Viruspartikeln, im Vergleich zum Kontrollansatz (Lösungsmittelkontrolle ohne MEK-Inhibitor U0126) kommt. Die makroskopische Untersuchung von MDCK Zellen, welche mit entsprechenden Konzentrationen an DMSO, bzw. in DMSO gelöstem MEK-Inhibitor U0126 behandelt wurden, als auch eine Zytotoxizitätsuntersuchung mittels Propidiumjodidfärbung, zeigt, daß weder Lösungsmittel noch Inhibitor einen signifikanten zytotoxischen Effekt auf die Zellen haben.

### Beispiel 3: Spezifische Hemmung der Virusvermehrung bei dem zellulären MEKK-SEK-JNK-Signalübertragungsweg

Der Einfluss einer spezifischen Hemmung von SEK/MKK4 wurde zunächst mit Hilfe der transienten Transfektion von MDCK-Zellen mit einer dominant negativen Mutante der Kinase SEK KD untersucht. Bei dieser Mutanten wurde ein Lysin-Aminosäurerest an Aminosäureposition 129 durch gezielte Mutagenese auf DNA-Ebene in einen Arginin-Aminosäurerest umgewandelt (Ludwig et al. Mol Cell Biol 16,6687-6697 1996), eine Mutation, welche die ATP-Bindung der Kinase stört und die Kinase somit inaktiv vorliegt. Diese Mutante wirkt bei Überexpression in der Zelle dominant negativ über den endogenen Wildtyp (Ludwig et al Mol Cell Biol 16,6687-6697 1996). Für das Experiment wurden MDCK-Zellen mit dem Leervektor pEBG oder dem Expressionskonstrukt pEBG SEK KD (Ludwig et al.Mol Cell Biol 16,6687-6697 1996) mit Hilfe des Transfektionsreagenz Lipofectamine 2000 (Life Technologies) nach Standardmethoden (Ludwig et al. J Biol Chem 276,10990-10998,2001) transfiziert. Die Transfektionseffizienzen lagen bei über 60%. 24 Stunden nach Transfektion erfolgte eine Infektion mit dem Influenza A Virusstamm fowl plage virus A/Fpv/Bratislava/79 mit einer Multiplizität der Infektion von 1 (M.O.I.=1). Weitere 24 Stunden nach Infektion wurden die Titer der neu gebildeten Viren im Zellkulturüberstand in Standard Plaque-Assays auf MDCK-Zellen überprüft. Verglichen wurden die Virustiter von Influenza A Virus-infizierten MDCK-Zellen, welche entweder mit dem Leervektor oder einem Konstrukt, welches die dominant negative Form von SEK exprimierte, infiziert worden waren. Desweiteren wurden mit Hilfe retroviraler Transduktion MDCK-Zelllinien hergestellt, welche stabil entweder SEK KD oder eine SEK antisense-RNA exprimierten. Eine Überexpression der dominant negativen Kinase SEK KD hemmt kompetitiv den endogenen Wildtyp, während durch Bildung einer zur SEK/MKK4 Boten-RNA komplementären RNA-Spezies (antisense RNA) die Synthese von endogener SEK/MKK4 inhibiert wird. Zur Generierung dieser stabilen MDCK-Zellinen wurde die cDNA für SEK KD sowohl in sense- als auch in antisense-Orientierung in den retroviralen Expressionsvektor pCFG5 IEGZ (Kuss et al. Eur J Immunol 29,3077-3088, 1999) einkloniert. Neben der Boten-RNA für SEK KD bzw. der antisense RNA, kodiert die Vektor-DNA weiterhin für die Boten-RNA des "green fluorescent protein" (GFP), welches während der Proteinsynthese ausgehend von einer internen Ribosomenbindestelle exprimiert wird. Dies erlaubt die Identifizierung stabil transduzierter Zellen in der Durchflusszytometrie. Darüber hinaus vermittelt der Vektor eine Resistenz gegen das Antibiotikum Zeocin. Die Expressionskonstrukte für SEK KD und SEK-antisense RNA sowie der Leervektor wurden mit Hilfe der Calciumphosphatprezipitationsmethode in die virusproduzierende Zelline ØNX (Grignani et al. Cancer Res 58,14-19,1998) transfiziert. Die Transfektionseffizienz wurde nach 24 Stunden anhand der GFP-Expression kontrolliert und befand sich in einer Größenordnung von 70-80%. Die Zellen wurden darauf hin für ca. zwei Wochen mit 1mg/ml Zeocin im Medium selektioniert. Zur Infektion von MDCK-Zellen mit den rekombinanten Retroviren wurden die retrovirushaltigen Mediumüberstände der virusproduzierenden Zellinien filtriert, mit 5 µg/ml Polybrene (Sigma-Aldrich, Taufkirchen b. München, Deutschland) versetzt und auf frische MDCK-Zellen gegeben. Die Infektion erfolgte während zweimal 3-stündiger Zentrifugation (1000xg) an zwei aufeinander folgenden Tagen. Stabil transduzierte MDCK-Zellen wurden 24 Stunden nach Infektion für zwei weitere Wochen mit 400-600 µg/ml Zeozin im Mediumüberstand selektioniert. Nach der so erfolgten Generierung der stabilen Zellinien wurden sowohl diese als auch Wildtyp-MDCK-Zellen mit Influenza A Virus infiziert und die Virustiter von SEK KD und SEK antisense-Linien wie oben beschrieben im Vergleich zum Titer aus dem Überstand von Wildtyp-MDCK-Zellen bestimmt. Folgende Ergebnisse wurden erhalten. Der Vergleich der Virustiter von Influenza A Virus-infizierten MDCK-Zellen, welche zuvor entweder mit dem Leervektor oder einem Konstrukt, das die dominant negative Form von SEK exprimierte, transfiziert worden sind, zeigte, dass in SEK KD exprimierenden Zellen die Vermehrung der Viren nach 24 Stunden zu mehr als 90% gehemmt war. Dieses Ergebnis war in mehreren unabhängigen Ansätzen reproduzierbar. Zur weiteren Untersuchung wurden mit Hilfe retroviraler Transduktion MDCK-Zelllinien hergestellt, welche stabil entweder SEK KD oder eine SEK antisense RNA exprimierten (siehe oben). In dem einen Fall wird die SEK/MKK4-Akti- vität durch Kompetition gehemmt, im anderen Falle erfolgt Expressionshemmung der Kinase. Auch in diesen Zelllinien waren die Virustiter 24 Stunden nach Infektion mit zwei verschiedenen Influenza A-Virusstämmen (FPV und WSN-HK) im Vergleich zu Wildtyp-Zellen stark reduziert, was belegt, dass die Blockierung des virus-aktivierten Signalübertragungsweges auf der Ebene von SEK eine entscheidenden Effekt auf die Virusvermehrung hat. Diese Befunde zeigen, dass die spezifische Inhibition der übergeordneten SEK Kinase innerhalb des zellulären MEKK/SEK/JNK-Signalübertragungsweges die Virusvermehrung hemmt, und zwar mindestens so effektiv wie der Kinaseinhibitor U0126, von welchem bekannt ist, dass er durch Hemmung von MEK die Signalübertragung über Raf-MEK-ERK inhibiert. Darüber hinaus zeigt die Möglichkeit, SEK KD und SEK antisense RNA überproduzierende Zellinien zu erzeugen, welche in Bezug auf ihre Morphologie und ihr Wachstumsverhalten nicht von Vektor-Zellen oder Wildtyp-MDCK-Zellen zu unterscheiden sind, dass die spezifische Hemmung von Funktion oder Expression dieser Kinase nicht toxisch für die Wirtszelle ist.

### Beispiel 4: Kombination zweier Wirksubstanzen zur Hemmung der Virusvermehrung bei dem zellulären Raf/MEK/ERK-Signalübertragungsweg

Der spezifische Effekt der Inhibition zweier Kinasen innnerhalb eines zellulären Signalübertragungsweges, die unmittelbar nacheinander aktivierbar sind, auf die Influenza Virus-Vermehrung, wurde am Beispiel des Raf/MEK/ERK-Signalübertragungsweges untersucht. Hierzu wurde mit Hilfe der transienten Transfektion zum einen eine dominant negative Mutante der Kinase Raf (RafC4B) und zum anderen dominant negative Mutanten der Kinase ERK2 (ERK2C3, ERK2B3) zur Hemmung der jeweiligen Wildtyp Kinase in MDCK-Zellen eingebracht und überexprimiert. RafC4B ist eine Deletionsmutante von Raf, der die Kinasedomäne fehlt (Bruder et al Genes Dev 6:545-556, 1992.). Damit wird das aktiviernde Signal auf der Ebene von Raf unterbrochen. ERK2B3 ist eine Punktmutante der Kinase ERK2, bei der ein konserviertes Lysin in der ATP-Bindestelle an Aminosäureposition 52 des Proteins durch gezielte Mutagenese auf DNA-Ebene gegen eine Arginin-Aminosäurerest umgewandelt ist, eine Mutation, welche die ATP-Bindung der Kinase stört und die Kinase somit inaktiv vorliegt (Robbins et al J Biol Chem 268:5097-5106, 1993). Bei ERK2C3 wurde ein Tyrosin-Aminosäurerest an Position 185 des Proteins im Sequenzmotiv Threonin-Glutaminsäure-Tyrosin, welches im Zuge der Aktivierung der Kinase phosphoryliert wird, in Phenylalanin umgewandelt, wodurch die Kinase nicht mehr aktiviert werden kann (Robbins et al J Biol Chem 268:5097-5106, 1993). ERK2C3 wirkt daher bei Überexpression entsprechend kompetitiv hemmend auf den endogenen Wildtyp. Für das Experiment wurden MDCK-Zellen mit dem Leervektor KRSPA oder mit den Expressionskonstrukten KRSPA RafC4B, KRSPA ERK2B3 oder KRSPA ERK2C3 mit Hilfe des Transfektionsreagenz Lipofectamine 2000 (Life Technologies/*Invitrogen*, *Karlsruhe, Deutschland)* nach Standardmethoden (Ludwig et al. J Biol Chem 276,10990-10998,2001) transfiziert. Die Transfektionseffizienzen lagen bei über 60%. 24 Stunden nach der Transfektion wurden bei einem Teil der Ansätze die Zellen zur zusätzlichen Hemmung von MEK in dem Signalübertragungsweg mit 30µM U0126, wie bereits vorangehend beschrieben, behandelt (Pleschka et al. Nat. Cell Biol. 3, 301-305, 2001). Danach erfolgte bei allen Ansätzen Infektion mit dem Influenza A Virusstamm fowl plage virus A/Fpv/Bratislava/79 (H7N7) mit einer Multiplizität der Infektion von 1 (M.O.I.=1). Weitere 9 Stunden bzw. 24 Stunden nach der Infektion wurden die Titer der neu gebildeten Viren im Zellkulturüberstand in Standard Plaque-Assays auf MDCK-Zellen hin überprüft. Verglichen wurden die Virustiter von: 1. infizierten MDCK-Zellen, welche mit dem Leervektor transfiziert waren, 2. infizierten MDCK-Zellen, welche entweder mit RafC4B, ERK2B3 oder ERK2C3 transfiziert waren, 3. infizierten MDCK-Zellen, welche mit dem Leervektor transfiziert und zusätzlich U0126 behandelt waren und 4. infizierten MDCK-Zellen, welche entweder mit RafC4B, ERK2B3 und ERK2C3 transfiziert und zusätzlich U0126 behandelt waren. Die folgenden Ergebnisse wurden erhalten. Der Vergleich der Virustiter von Influenza A Virus-infizierten MDCK-Zellen, welche zuvor entweder mit dem Leervektor oder Konstrukten, welche dominant negative Formen von RafC4B, ERK2B3 oder ERK2C3 exprimierten, transfiziert worden waren, zeigte, dass in Zellen, welche die Kinasemutanten exprimierten, die Vermehrung der Viren nach 9 Stunden und 24 Stunden signifikant gehemmt war. Das gleiche gilt für die Virusvermehrung in Zellen, welche mit Leervektor transfiziert und mit U0126 behandelt wurden im Vergleich zu nicht U0126 behandelten Zellen. Wird in Zellen, in welchen Raf oder ERK durch Überexpression von RafC4B bzw. ERK2B3 oder ERK2C3 gehemmt wurde, erfindungsgemäß zusätzlich die Kinase MEK als zweite Kinase innerhalb des Signalübertragungsweges gehemmt, findet man eine verstärkte Hemmung der Virusvermehrung im Vergleich zu den Ansätzen, die keine zusätzliche Hemmung der Kinase MEK durch U0126 erfahren haben. Dies zeigt, dass die Hemmung zweier Kinasen innerhalb eines Signalübertragungsweges die Virusvermehrung in stärkerem Maße inhibiert, als dies durch Hemmung nur einer Kinase innerhalb des Signalübertragungsweges möglich ist.

### Beispiel 5: Spezifische Hemmung der Virusvermehrung bei dem zellulären Raf/MEK/ERK-Signalübertragungsweg

Der spezifische Effekt der Inhibition von Kinasen innnerhalb eines zellulären Signalübertragungsweges, auf die Influenza Virus-Vermehrung, wurde am Beispiel des Raf/MEK/ERK-Signalübertragungsweges untersucht. Hierzu wurde mit Hilfe der transienten Transfektion zum einen eine dominant negative Mutante der Kinase Raf (RafC4B) und zum anderen dominant negative Mutanten der Kinase ERK2 (ERK2C3, ERK2B3) (s. o.) zur Hemmung der jeweiligen Wildtyp Kinase in MDCK-Zellen eingebracht und überexprimiert. Für das Experiment wurden MDCK-Zellen mit dem Leervektor KRSPA oder mit den Expressionskonstrukten KRSPA RafC4B, KRSPA ERK2B3 oder KRSPA ERK2C3 mit Hilfe des Transfektionsreagenz Lipofectamine 2000 (Life Technologies/Invitrogen, *Karlsruhe, Deutschland)* nach Standardmethoden (Ludwig et al. J Biol Chem 276,10990-10998,2001) transfiziert. Die Transfektionseffizienzen lagen bei über 60%. Nach 24 Stunden erfolgte bei allen Ansätzen Infektion mit dem Influenza A Virusstamm fowl page virus A/Fpv/Bratislava/79 (H7N7) mit einer Multiplizität der Infektion von 1 (M.O.I.=1). Weitere 9-24 Stunden nach der Infektion wurden die Titer der neu gebildeten Viren im Zellkulturüberstand in Standard Plaque-Assays auf MDCK-Zellen hin überprüft. Verglichen wurden die Virustiter von: 5. infizierten MDCK-Zellen, welche mit dem Leervektor transfiziert waren, 6. infizierten MDCK-Zellen, welche entweder mit RafC4B, ERK2B3 oder ERK2C3 transfiziert waren. Die folgenden Ergebnisse wurden erhalten. Der Vergleich der Virustiter von Influenza A Virus-infizierten MDCK-Zellen, welche zuvor entweder mit dem Leervektor oder Konstrukten, welche dominant negative Formen von RafC4B, ERK2B3 oder ERK2C3 exprimierten, transfiziert worden waren, zeigte, dass in Zellen, welche die Kinasemutanten exprimierten, die Vermehrung der Viren signifikant gehemmt war. Dies zeigt, dass die Hemmung verschiedener Kinasen innerhalb eines Signalübertragungsweges die Ansatzpunkte zur Inhibition der Virusvermehrung darstellen können.

### Beispiel 6: Kombination zweier Wirksubstanzen zur Hemmung der Virusvermehrung bei dem zellulären MKK6/p38/3pK-Signalübertragungsweg

Die spezifische Wirkung der Inhibition zweier Kinasen innnerhalb eines zellulären Signalübertragungsweges, die unmittelbar nacheinander aktivierbar sind, auf die Influenza Virus-Vermehrung wurde ferner am Beispiel des zellulären MKK6/p38/3pK-Signalübertragungsweges untersucht. Hierzu wurde mit Hilfe der transienten Transfektion zum einen eine dominant negative Mutante der Kinase MKK6 (MKK6(Ala)) und zum anderen eine dominant negative Formen der Kinase 3pK (3pK K>M), ein Kinasesubstrat der p38 MAP-Kinase, zur Hemmung der jeweiligen Wildtyp-Kinase in MDCK-Zellen eingebracht und überexprimiert. 3pK K>M ist eine Punktmutante der Kinase 3pK, bei der ein konserviertes Lysin in der ATP-Bindestelle an Aminosäureposition 73 des Proteins durch gezielte Mutagenese auf DNA-Ebene gegen ein Methionin umgewandelt wurde (Sithanandam et al. Mol Cell Biol 16:868-876,1996), eine Mutation, welche die ATP-Bindung der Kinase stört und die Kinase somit inaktiv vorliegt. Bei MKK6(Ala) wurde entsprechend das Lysin in der ATP-Bindestelle an Aminosäureposition 82 des Proteins durch gezielte Mutagenese auf DNA-Ebene in ein Alanin umgewandelt (Raingeaud et al Mol Cell Biol 16:1247-1255, 1996). Durch diese Inaktivierung wirkt MKK6(Ala) bei Überexpression kompetitiv hemmend auf den endogenen Wildtyp. Für das Experiment wurden MDCK-Zellen mit dem Leervektor KRSPA oder mit den Expressionskonstrukten KRSPA MKK6(Ala) oder 3pK K>M mit Hilfe des Transfektionsreagenz Lipofectamine 2000 (Life Technologies) nach Standardmethoden (Ludwig et al. J Biol Chem 276,10990-10998,2001) transfiziert. Die Transfektionseffizienzen lagen bei über 60%. 24 Stunden nach Transfektion wurden bei einem Teil der Ansätze die Zellen zur zusätzlichen Hemmung der Kinase in dem Signalübertragungsweg mit 20µM SB202190, einem spezifischen Inhibitor der p38 MAP-Kinase (Cohen Trends Cell Biol 7:353-361, 1997) behandelt. Danach erfolgte bei allen Ansätzen Infektion mit dem Influenza A-Virusstamm fowl plage virus A/Fpv/Bratislava/79 mit einer Multiplizität der Infektion von 1 (M.O.I.=1). Weitere 9 Stunden bzw. 24 Stunden nach Infektion wurden die Titer der neu gebildeten Viren im Zellkulturüberstand in Standard Plaque-Assays auf MDCK-Zellen hin überprüft. Verglichen wurde die Virustiter von: 1. infizierten MDCK-Zellen, welche mit dem Leervektor transfiziert waren, 2. infizierten MDCK-Zellen, welche entweder mit MKK6(Ala) oder 3pK K>M transfiziert waren, 3. infizierten MDCK-Zellen, welche mit dem Leervektor transfiziert und zusätzlich SB202190 behandelt waren und 4. infizierten MDCK-Zellen, welche entweder mit MKK6(Ala) oder 3pK K>M transfiziert und zusätzlich mit SB202190 behandelt worden waren. Die folgenden Ergebnisse wurden erhalten. Der Vergleich der Virustiter von Influenza A Virus-infizierten MDCK-Zellen, welche zuvor entweder mit dem Leervektor oder mit Konstrukten, welche dominant negative Formen von MKK6 oder 3pK exprimierten, transfiziert worden waren, zeigte, dass in Zellen, welche diese Kinasemutanten exprimierten, die Vermehrung der Viren nach 9 Stunden und 24 Stunden signifikant gehemmt war. Das gleiche gilt für die Virusvermehrung in Zellen, welche mit Leervektor transfiziert und mit SB202190 behandelt wurden im Vergleich zu nicht SB202190 behandelten Zellen. Wird in Zellen, in welchen MKK6 oder 3pK durch Überexpression von MKK6(Ala) oder 3pK K>M gehemmt wurde, erfindungsgemäß zusätzlich die Kinase p38 als zweite Kinase innerhalb des Signalübertragungsweges gehemmt, findet man eine verstärkte Hemmung der Virusvermehrung im Vergleich zu den Ansätzen, die keine zusätzliche Hemmung der Kinase p38 durch SB202190 erfahren haben. Dies zeigt, dass die Hemmung zweier Kinasen innerhalb eines zellulären Signalübertragungsweges die Virusvermehrung in stärkerem Maße inhibiert, als dies durch Hemmung nur einer Kinase innerhalb des Signalübertragungsweges möglich ist.

### Beispiel 7: Wirkung der Kombination von Amantadin und U 0126 auf die Influenza Virus Infektion

Von Scholtissek und Müller (Arch. Virol. 119:111-118,1991) wurde gezeigt, dass die Behandlung von Influenza infizierten Zellen mit einer Kombination eines Methylierungs-hemmers (3-Deaza-adenosin) und eines breit wirkenden toxischen Kinase-Hemmers (1-(5-Isoquino-Line-sulphonyl)- 2-Methylpiperazin = H7) die Wirkung der beiden einzelnen Inhibitoren potenziert. Geprüft wurde, ob die Kombination einer erfindungsgemäßen Wirksubstanz mit einer antiviralen Substanz, welche eine virale Komponente hemmt und kein Kinasehemmer ist, eine synergistische antivirale Wirksamkeit aufweist. Permissive MDCK-Zellen wurden mit Multiplizität der Infektion (MOI) von 0,01 mit Influenza A Viren (A/FPV/Bratislava (H7N7) und A/WSN-HK (H3N1)) und Influenza B Viren/Massachusetts/6/92 (B/Mass) infiziert. Die infizierten Zellen wurden wie folgt behandelt: 1) unbehandelt (für FPV, WSN-HK und B/ Mass), 2) U 0126 in optimal antiviral wirkenden Konzentrationen (Pleschka et al Nature Cell Biol 3: 301-305,2001) (für FPV, WSN-HK und B/ Mass), 3) Amantadine in optimal antiviral wirkenden Konzentrationen (Hay et al EMBOJ 11: 3021-3024,1985) (für FPV), 4) Kombinationen von Amantadine und U0126 (für FPV). Von Amantadine ist bekannt, i) dass es in pharmakologisch sinnvollen (mikromolaren) Konzentrationen nur bei Influenza A Infektionen, nicht jedoch bei Influenza B Infektionen wirkt (Davies et al Science 144: 862-863,1964), ii) dass es nicht bei allen Influenza Viren des Subtyps A wirkt, z.B. nicht bei A/WSN/33 (Thomas et al J.Virol. 252,54-64,1998) oder bei A/Puerto Rico/8/34 (Castrucci et al J.Virol 69:2725-8,1995), iii) dass es sensitive Influenza A Viren anfänglich stark hemmt, nachfolgend zur Bildung von resistenten Virusvarianten führt (Hay et al EMBO J. 11: 3021 -3024,1985). 48 Stunden nach der Infektion wurde der Titer an infektiösen Viruspartikeln im Zellkulturüberstand bestimmt. Die Überstände von unbehandelten und von mit Amantadine und/oder mit U0126 behandelten Zellen wurden 1: 1000 verdünnt (die Überstände von Influenza A/WSN-HK und Influenza B Virus infizierten Zellen, die mit U0126 behandelt worden waren, wurden 1:100 verdünnt) und erneut zur Infektion von frischen Zellen benutzt. Diese Infektionsrunden wurden zweimal wiederholt. Folgendes Ergebnis wurde erzielt: i) schon nach der zweiten Infektionsrunde waren bei Behandlung mit optimalen Konzentrationen von U0126 eine deutliche Verminderung der Influenza-Virus-Partikel zu verzeichnen, die bei einem Versuch soweit ging, dass nicht mehr genügend infektiöse Influenza A/WSN-HK oder Influenza B Virus Partikel vorhanden waren, um eine dritte Infektionsrunde zu ermöglichen, ii) der Influenza A /FPV VirusTiter blieb bei Behandlung mit einer optimalen Konzentration von U0126 gleichbleibend niedrig, obwohl sich dieser Virussubtyp wesentlich stärker in Zellkultur vermehrt als beispielsweise Influenza A/WSN-HK, iii) der Titer des Amantadinesensitiven Influenza A/FPV Virus nach Zugabe von Amantadin abfiel und nachfolgend wieder anstieg, was auf die Bildung resistenter Virusvarianten hinweist, iv) der Titer des nicht Amantadine -resistenten Influenza A/WSN_HK Virus trotz der Amantadine-Behandlung anstieg, was bestätigt, dass nicht alle Influenza A Viren auf Amantadine reagieren, v) die Behandlung mit Amantadine und einer optimal antiviral wirkenden Konzentration von U0126 bei FPV zu einer verstärkten Hemmung der Virusvermehrung führt. Die Ergebnisse zeigen somit, die überlegene antivirale Wirkung eines erfindungsgemäßen Wirkstoffes, welcher sowohl gegen verschiedene Influenza A Viren (welche teilweise Amantadin Resistent sind) als auch gegen Influenza B Viren (welche Amantadin resistent sind) wirkt, wobei keine Resistenzbildung weder bei Influenza A Viren noch bei Influenza B Viren beobachtet wurde, und der eine synergistische Wirkung in der Kombination eines erfindungsgemäßen Wirkstoffes mit einem antiviral wirkendem Wirkstoff, der kein Kinaseinhibitor ist auf Influenza A Viren zeigt.

### Beispiel 8: Resistenzbildung

Dieses Beispiel zeigt, daß unter der Wirkung des MEK-Inhibitors U0126 bei mehrfacher Passagierung keine resistente Varianten von Influenza A und B Viren bilden. Für die Vermehrung von Influenza A und B Viren werden permissive, eukaryontische Zellkulturen (Madine darby canine kidney (MDCK)-Zellen), in parallelen Ansätzen mit gleicher Zellzahl, nach allgemein üblichen Methoden für die Zellkultur, mit einer physiologischen Salzlösung gewaschen und mit einer Menge des infektiösen Influenza A Virusstammes fowl plage virus A/Fpv/Bratislava/79 (H7N7) bzw. des infektiösem Influenza B Virusstammes Massachusetts/6/93, in einem Verhältnis von 0,01 infektiöse Viruspartikel pro Zelle für eine Stunde bei Raumtemperatur, infiziert. Nach der Infektion wird das Inokulum entfernt und die infizierten Zellen in geeignetem Zellkulturmedium, welches mit dem MEK-Inhibitor U0126 versetzt sind (50µM gelöst in DMSO), für 48h, bei 37°C und 5% CO₂-Konzentration, inkubiert. Als Lösungsmittelkontrolle werden infizierte MDCK-Zellen mit Zellkulturmedium inkubiert, welches mit der entsprechenden Mengen an DMSO versetzt ist. Für die Influenza B Virusinfektion enthält das Zellkulturmedium 2µg/ml Trypsin. Für die Influenza A Virusinfektion wurde der Zellüberstand nach 48 Stunden geerntet. Für die Influenza B Virusinfektion wurde 48 Stunden nach der Infektion die gleiche Menge an Inhibitor bzw. Lösungsmittel erneut zugegeben. Der Zellüberstand wurde nach weiteren 24 Stunden geerntet. 0,1 ml einer 10-2 Verdünnung (Influenza B Virus) bzw. eine 10-3 Verdünnung (Influenza A Virus) der Zellüberstände wurde zur Infektion von frischen MDCK-Zellen benutzt (Passage). Das Protokoll wurde vier mal wiederholt und nach jedem Durchgang der Virustiter bestimmt. Die Proben der jeweiligen Zellkulturüberstände werden nach üblichen virologischen Methoden auf die Menge an neu gebildeten infektiösen Viruspartikeln (Plaque-Assay auf MDCK-Zellen) hin untersucht. In einer zusätzlichen Kontrolle wurden FPV infizierte MDCK-Zellen mit Amantadin (5µM, Zellüberstände wurden 48 Stunden nach der Infektion geerntet und 0,1ml einer 10-3 Verdünnung zur Passagierung verwendet) behandelt, welches die Vermehrung von einigen Influenza A Viren signifikant hemmt, da es die Ionenkanalaktivität des M2-Proteins hemmt. Zu Amantadine wird auf das Beispiel 7 verwiesen. Im Ergebnis kann in einem solchen experimentellen Ansatz festgestellt werden, daß es bei der entsprechenden Konzentration des MEK-Inhibitors U0126 im Zellkulturmedium zu einer signifikanten und konstanten Reduktion (ca. 80%) der Anzahl von neu gebildeten, infektiösen Influenza A Viruspartikeln, im Vergleich zur Lösungsmittelkontrolle ohne MEK-Inhibitor U0126 kommt, ohne das sich resistente Virusvarianten bilden, welche zu einem Anstieg des Virustiters führen würden, wie es im Fall der Amantadinbehandlung deutlich zu erkennen ist. Des weiteren erkennt man, daß es ebenfalls zu einer signifikanten und konstanten Reduktion (ca. 90%) der Anzahl von neu gebildeten, infektiösen Influenza B Viruspartikeln, im Vergleich zur Lösungsmittelkontrolle ohne MEK-Inhibitor U0126 kommt ohne das sich resistente Virusvarianten bilden, welche zu einem Anstieg des Virustiters führen würden.

### Beispiel 9: Negativkontrolle

Dieses Beispiel zeigt, daß unter der Wirkung der transienten Expression konstitutiv aktiver Formen der Kinasen Raf (Raf BXB, Deletionsmutante, die nur noch die Kinasedomäne enthält, beschreiben in: Flory, E., Weber, C. K., Chen, P., Hoffmeyer, A., Jassoy, C. & Rapp, U. R. (1998) J. Virol. 72, 2788-2794) und MEK (delta Stu MEK, entstand durch Stu vermittelte Deletion einer inhibitorischen alpha-Helix, beschreiben in: Apoptosis suppression by Raf-1 and MEK1 requires MEK- and phosphatidylinositol 3-kinase-dependent signals.von Gise A, Lorenz P, Wellbrock C, Hemmings B, Berberich-Siebelt F, Rapp UR, Troppmair J. Mol Cell Biol 2001 Apr;21(7):2324-36) in MDCK-Zellen die Vermehrung von Influenza A Virus signifikant gesteigert wird. Für die Vermehrung von Influenza A Viren werden permissive, eukaryontische Zellkulturen MDCK-Zellen, in parallelen Ansätzen mit gleicher Zellzahl, nach allgemein üblichen Methoden für die Zellkultur, mit Plasmid-DNA der entsprechenden Expressionsplasmide transfiziert bzw. der Vektorkontrolle (s. o). Die Transfektionseffizienzen lagen hierbei ca. 80%. 24 Stunden nach der Transfektion werden die Zellen nach allgemein üblichen Methoden für die Zellkultur, mit einer physiologischen Salzlösung gewaschen und mit einer Menge des infektiösen Influenza A Virusstammes fowl plage virus A/Fpv/Bratislava/79 (H7N7), in einem Verhältnis von 1-10 infektiöse Viruspartikel pro Zelle für eine Stunde bei Raumtemperatur, infiziert. Anschließend wird das Inokulum durch geeignetes Zellkulturmedium ersetzt. Nach weiteren 24 Stunden wurde die Zahl neugebildeter infektiöser Viruspartikel durch Plaque Assay bestimmt (s. O.). Im Ergebnis kann in einem solchen experimentellen Ansatz festgestellt werden, daß die Zahl neugebildeter, infektiöser Viruspartikel von MDCK-Zellen, die vor der Virusinfektion mit den entsprechenden Expressionplasmiden transfiziert wurden waren, im Vergleich zu MDCK-Zellen, die vor der Virusinfektion nur mit der Vektorkontrolle transfiziert wurden waren, für Influenza A Viren signifikant ansteigt.

## Patentansprüche

1. Kombinationspräparat zur Prophylaxe und/oder Therapie von mindestens einer Viruserkrankung, enthaltend mindestens eine Wirksubstanz, welche entweder mindestens zwei Kinasen oder mindestens eine SEK Kinase eines zellulären Signalübertragungsweges derart hemmt, dass eine Virusvermehrung gehemmt wird, und mindestens eine antiviral wirkende Substanz, die kein Kinaseinhibitor ist.

2. Kombinationspräparat nach Anspruch 1,
wobei die Kinasen des zellulären Signalübertragungsweges unmittelbar nacheinander aktivierbar sind, und/oder
wobei die Kinasen bzw. die SEK Kinase des zellulären Signalübertragungsweges aus nachfolgenden Signalübertragungswegen ausgewählt sind bzw. ist: MEKK2,-3/MEK5/ERK5; Raf/MEK/ERK; MEKK/SEK/JNK; JAK1, JAK2, JAK3, TYK2 und/oder Hetero- und Homodimere von JAK1,-2,-3,TYK2; ASK/MKK3,-6/p38; ASK/MKK4,-7/JNK; MEKK4/MKK4,-7; DLK/MKK4,-7; Tpl-2/MKK4,-7; Tpl-2/MEK5/ERK5; MLK-3/ MKK3,-6; MLK-3/ MKK4,-7; TAK/NIK/IKK; TAK/MKK3,-6; TAK/MKK4,-7; PAK/MKK3,-6; PAK/IKK; Cot,Tpl-2/IKK; PKC/IKK; PKB/IKK; PKC/Raf; PAK/Raf; Lck/Raf; MEKKs/IKK; PI3K/PDK1/PKB; JAK/TYK/PLCgamma; und/oder MAP Kinasen/MAPKAP-Kinasen, beispielsweise ERK/3pK, ERK/Rskp90, p38/MAPKAP-Kinasen und/oder p38/3pK, und/oder
wobei die Wirksubstanz(en) aus nachfolgenden Wirksubstanzen ausgewählt ist bzw. sind: Kinase-inhibierendes Flavon-Derivat oder Benzpyran-Derivat; Kinase-inhibierendes Derivat des 4H-1-Benzopyran; Flavopiridolderivat; 2-(2-Amino-3-methoxyphenyl)-4-oxo-4H-(1) benzopyran; 7,12-Dihydro-indolo (3,2-d)(1) Benzazepin-6(5H)-on; 70H-Staurosporin und/oder ein Phosphokinase-inhibierendes Derivat des 70H-Staurosporin; Butyrolacton; Roscovitine; Purvalanol A; Emodin; Anilinoquin-azolin; Phenylaminopyrimidin; Trioylimidazol; Paullon; [4-(4-Fluorophenyl)-2-(4-methylsulfinylphenyl)-5-(4-pyridyl)1H-imidazol; [1,4-Diamino-2,3-dicyano-1,4-bis (2aminophenylthio) butadien; Kinase-inhibierendes Derivat des Butadien; [2-2'-Amino-3'-methoxyphenyl)-oxanaphtalen-4-on); [2-(2-Chlor-4-jod-phenylamino)-N-cyclopropylmethoxy-3,4-difluor benzamid; CEP-1347 (KT7515) bisethylthiomethyl; Tetrapyrrolisches Makrocyclen; Pyrimidonderivat; 3-Aminomethylen-indolin-Derivat; Pyrazolo (3,4-b) pyridin-Derivat; Pyrazol-Derivat;1,4-substituiertes Piperidinderivat; lipoides Ammoniumsalz; dominant negative Mutante einer Kinase eines zellulären Signalübertragungsweges; Antisense-Oligonukleotid, welches sich spezifisch an die DNA-Sequenz oder mRNA-Sequenz kodierend für eine Kinase eines zellulären Signalübertragungsweges anlagert und deren Transkription bzw. Translation hemmt; dsOligonukleotide, die geeignet sind zur gezielten Degradierung der mRNAs von Kinasen eines zellulären Signalübertragungsweges durch RNAiTechnologie; Antikörper oder Antikörperfragment, spezifisch für eine Kinase oder ein Fusionsprotein, enthaltend mindestens ein Antikörperfragment, beispielsweise ein Fv-Fragment, das die Kinaseaktivität eines Kinasemoduls hemmt; und/oder Peptid, das die Interaktion von mindestens zwei, vorzugsweise unmittelbar nacheinander aktivierbarer, Kinasen eines zellulären Signalübertragungsweges hemmt.

3. Kombinationspräparat zur Prophylaxe und/oder Therapie von mindestens einer Viruserkrankung, enthaltend mindestens zwei Wirksubstanzen, welche entweder auf mindestens zwei Kinasen eines zellulären Signalübertragungsweges derart einwirken, dass eine Virusvermehrung im Wesentlichen gehemmt wird oder eine SEK-Kinase im Wesentlichen hemmen, vorzugsweise ausgewählt aus den Wirksubstanzen gemäß Anspruch 2, wobei das Kombinationspräparat in Form eines Gemisches oder als einzelne Komponenten zur gleichzeitigen oder zeitlich unterschiedlichen Anwendung an gleichen oder unterschiedlichen Orten anwendbar ist.

4. Kombinationspräparat nach einem der Ansprüche 1 oder 2, wobei die antiviral wirkende Substanz, welche kein Kinaseinhibitor ist , 1-Adamantanamin, Rimantadine, ein Neuraminidaseinhibitor oder ein Nukleosidanalogon, wie Ribavirin, ist.

5. Verwendung eines Kombinationspräparates nach einem der Ansprüche 1 oder 2 zur Herstellung einer pharmazeutischen Zusammensetzung für die Prophylaxe und/oder Therapie einer Infektion mit RNA- oder DNA Viren, vorzugsweise einer Infektion mit Negativstrang-RNA Viren, insbesondere Influenza Viren oder Bornaviren

6. Testsystem zum Auffinden von Wirksubstanzen, welche entweder auf mindestens zwei Kinasen oder auf eine SEK Kinase eines zellulären Signalübertragungsweges derart einwirken, dass eine Virusvermehrung im Wesentlichen gehemmt wird, enthaltend: a) mindestens eine mit mindestens einem Virus infizierbare Zelle, die entweder mindestens zwei Kinasen eines zellulären Signalübertragungsweges oder mindestens eine SEK Kinase enthält und mindestens einen die Zellen infizierenden Virus oder b) mindestens eine mit mindestens einem Virus infizierte Zelle, die entweder mindestens zwei Kinasen eines zellulären Signalübertragungsweges oder mindestens eine SEK Kinase enthält.

7. Testsystem nach Anspruch 6,
wobei es sich bei dem Virus um ein RNA- oder DNA-Virus, vorzugsweise um ein Influenza-Virus handelt, und/oder
wobei die Zelle mindestens eine überexprimierte Kinase enthält, und/oder
wobei es eine Zelle enthält, in welcher a) mindestens ein Gen kodierend für mindestens eine dominant negative Mutante mindestens einer übergeordneten Kinase und/oder b) mindestens eine dominant negative Mutante mindestens einer untergeordneten Kinase enthält, und/oder
wobei es eine Zelle enthält, in der die Expression für mindestens eine Kinase inhibiert ist.

8. Verfahren zum Auffinden von mindestens einer Wirksubstanz zur Prophylaxe und/oder Therapie von Viruserkrankungen, welche die Vermehrung von Viren bei Viruserkrankungen im Wesentlichen hemmt bzw. hemmen, enthaltend folgende Schritte: c) Inkontaktbringen mindestens eines Testsystems nach einem der Ansprüche 6 oder 7 mit mindestens einer potenziellen Wirksubstanz, und d) Bestimmung der Auswirkung auf die Virusvermehrung, wobei vorzugsweise ein prospektiver Wirkstoff, optional mit einem zuvor identifizierten verschiedenen Wirkstoff oder mit einem verschiedenen weiteren prospektiven Wirkstoff, mit einem Testsystem nach einem der Ansprüche 6 oder 7 kontaktiert wird, wobei die Hemmung der Virusvermehrung quantifiziert wird, wobei der erhaltene Wert der Hemmung mit einem Referenzwert der Hemmung verglichen wird, welcher unter gleichen Bedingungen, jedoch ohne einen prospektiven Wirkstoff oder jedoch mit einem Referenzwirkstoff oder einer Referenzwirkstoffkombination, gewonnen wird, und wobei ein prospektiver Wirkstoff oder eine prospektive Wirkstoffkombination selektiert werden, wenn der Wert der Hemmung höher als der Referenzwert der Hemmung ist.

9. Verfahren zur Herstellung eines Arzneimittels zur Prophylaxe und/oder Therapie von mindestens einer Viruserkrankung, welches die Vermehrung von Viren bei Viruserkrankungen im Wesentlichen hemmt bzw. hemmen, enthaltend folgende Schritte: c) Durchführen eines Testsystems nach einem der Ansprüche 6 oder 7, und d) Versetzen des/der aufgefundenen Wirksubstanz/en mit mindestens einem Hilfs- und/oder Zusatzstoff.

10. Verwendung eines oder mehrerer der folgenden Wirkstoffe zur Herstellung einer pharmazeutischen Zusammensetzung zur Prophylaxe oder Behandlung einer Viruserkrankung: a) ein Inhibitor einer Kinase, deren Inhibierung die Virusvermehrung hemmt, in Kombination mit einem antiviralen Wirkstoff, welcher kein Kinaseinhibitor ist, b) ein Inhibitor einer ersten Kinase, deren alleinige Inhibierung die Virusvermehrung hemmt, wobei der Inhibitor zusätzlich eine von der ersten Kinase verschiedene zweite Kinase, deren alleinige Inhibierung die Virusvermehrung hemmt, inhibiert, c) ein erster Inhibitor einer ersten Kinase, deren alleinige Inhibierung die Virusvermehrung hemmt, in Kombination mit einem vom ersten Inhibitor verschiedenen zweiten Inhibitor einer von der ersten Kinase verschiedene zweite Kinase, deren alleinige Inhibierung die Virusvermehrung hemmt, und/oder d) ein Inhibitor einer SEK Kinase.
